# EUROPÄISCHE PATENTANMELDUNG

(11) **EP 4 056 221 A1**
(43) Veröffentlichungstag der Anmeldung: **14.09.2022**
(21) Anmeldenummer: 21161563.8
(22) Anmeldetag: 09.03.2021
(51) Int. Cl.: A61M 25/00, A61M 29/00

(54) **SCHLAUCHVORRICHTUNG UND VERFAHREN ZUM SELEKTIVEN VERSTEIFEN EINER SCHLAUCHVORRICHTUNG**

(71) Anmelder: SoftRail Medical AG, 8500 Frauenfeld (CH)
(72) Erfinder: Gerig, Thomas, 3400 Burgdorf (CH); von Weymarn, Alexander, 8500 Frauenfeld (CH)
(74) Vertreter: Hepp Wenger Ryffel AG

(57) **Zusammenfassung**

Die Erfindung betrifft eine Schlauchvorrichtung (101), insbesondere Schleusenvorrichtung zum Einführen in einen Körpergang, die durch konzentrische Anordnung eines Innen (1)- und Aussenschlauchs (4), sowie einer Versteifungsschicht (3) durch Druckzufuhr reversibel versteift werden kann, indem die Versteifungsschicht Druck auf den Aussenschlauch ausübt und somit starke Reibung erzeugt.

## Beschreibung

Die Erfindung richtet sich auf eine Schlauchvorrichtung und ein Verfahren zum selektiven Versteifen einer Schlauchvorrichtung.

Bei minimal-invasiven operativen Verfahren, wie der Behandlung von Aneurysmen mit dem Legen von Stents, dilatieren, lysieren und embolisieren, kommen Schlauchvorrichtungen zum Einsatz, die über die Gefässe in den Körper eingeführt werden können.

In vielen Fällen müssen die Katheter, die oftmals über die Leistenarterie eingeführt werden, lange Strecken im Körper des Patienten zurücklegen und Windungen entlang der Blutgefässe vollführen können.

Ausserdem muss für die Behandlung ein Innenraum innerhalb des Katheters zum Einführen von Werkzeugen und/oder einem Führungsdraht und/oder operativen Behandlungselemente wie Stents, Coils oder ähnliches möglich sein.

Bei diesen medizinischen Behandlungen ist es besonders wichtig möglichst präzise und minimal-invasiv arbeiten zu können, da die Gesundheit des zu Behandelnden in Mitleidenschaft gezogen werden kann.

Da die Schlauchvorrichtung, z.B. ein Katheter, elastisch verformbar sein muss, um sich den Gefässwindungen anzupassen, gesteuert und eingeführt werden zu können, kann sie sehr leicht abrutschen oder abgelenkt werden. Dies ist zum einen sehr gefährlich, da somit nur schwer präzise gearbeitet werden kann und die Behandlung in die Länge gezogen werden kann. Zum anderen erhöht der flexible Katheter die Gefahr, dass der Patient Schaden erleidet oder sogar stirbt. Beispielsweise können sich ein Aneurysma, oder in den Gefässen vorhandene Kalkablagerungen, durch ungünstiges Verrutschen lösen und schwere Schäden verursachen. Vielfach muss auf einen anderen Katheter gewechselt werden, so dass der Materialverbrauch sehr hoch ist und somit auch die Kosten.

Es ist daher Gegenstand der vorliegenden Erfindung, diese Nachteile des Standes der Technik zu überwinden und eine Vorrichtung vorzustellen, mit der minimal-invasive operative Eingriffe mit möglichst geringem Risiko verbunden sind, schnell ausgeführt werden können und die Kosten gering gehalten werden können.

Die Aufgabe wird durch eine Schlauchvorrichtung gemäss der unabhängigen Ansprüche der Erfindung gelöst.

Eine Schlauchvorrichtung, insbesondere Schleusenvorrichtung, zum Einführen in einen Körpergang, umfasst einen Versteifungsabschnitt mit einem Innenschlauch, sowie eine Versteifungsschicht und einen Aussenschlauch. Die Versteifungsschicht ist konzentrisch ausserhalb des Innenschlauches und der Aussenschlauch konzentrisch ausserhalb der Versteifungsschicht angeordnet. Der Innenschlauch und der Aussenschlauch sind radial steif ausgebildet und die Versteifungsschicht radial bewegbar ausgebildet. Zwischen Innenschlauch und Versteifungsschicht ist derartig ein Druck aufbaubar, dass die Versteifungsschicht von innen gegen den Aussenschlauch drückbar ist und so unter Druck den Versteifungsabschnitt versteift.

Die Schlauchvorrichtung hat somit den Vorteil, dass sie während der Verwendung durch einen Druck reversibel versteift werden kann. Der bevorzugte Druck liegt im Wesentlichen in einem Bereich von 3-40 bar, insbesondere im Wesentlichen bei 16 bar, aber auch andere Druckbereiche sind vorstellbar, solange eine Versteifung gewährleistet ist.

Radial steif im Sinne der Erfindung heisst, dass die radial steife Schicht nur minimal durch Druck in radialer Richtung erweiterbar ist und so überhaupt ein Druck aufgebracht werden kann. Radial bewegbar heisst, dass die Schicht nicht radial steif ist. Radial steif schliesst nicht aus, dass die Schicht in anderen Richtungen flexibel ist.

Der Druck kann vorzugsweise durch eine externe Vorrichtung, die weitere Schutzmassnahmen beinhalten kann, wie beispielsweise das Abschalten bei Druckabfall durch ein Leck, aufgebracht überprüft und abgelassen werden, oder manuell angelegt werden.

An dieser Stelle ist anzumerken, dass die Druckdifferenz zum menschlichen Körper berücksichtigt werden muss. Deshalb ist die Schlauchvorrichtung so ausgebildet, dass ein hoher Druck zu- und abgeführt werden kann. Bevorzugt wird ein Druck oberhalb des atmosphärischen Druckes verwendet und kein Vakuum bzw. Unterdruck. Dies hat den Vorteil, dass die Druckdifferenz viel höher ausfällt, da der Unterdruck auf einen Maximalwert von 1 bar beschränkt ist.

Die Ausbildung einer Versteifung erleichtert das Fixieren der Schlauchvorrichtung an der gewünschten Stelle und erlaubt einen präziseren Eingriff, der die Sicherheit des Patienten erhöht, praktikabler durchführbar ist und ein schnelleres Arbeiten gewährleistet.

Im versteiften Zustand, kann der Druck zudem wieder reduziert werden und die Versteifung rückgängig gemacht werden.

Der versteifbar ausgebildete Bereich der Schlauchvorrichtung kann zudem in der Länge variieren, da die Versteifungsschicht nicht über die komplette Länge der Schlauchvorrichtung ausgebildet sein muss. Dementsprechend können bestimmte Bereiche der Schlauchvorrichtung versteifbar ausgebildet sein. Der Versteifungsabschnitt der Schlauchvorrichtung kann die vollständige Schlauchvorrichtung umfassen oder nur einen Teilabschnitt. Sofern nur ein Teilabschnitt der Schlauchvorrichtung durch den Versteifungsabschnitt gebildet wird ist der Rest der Schlauchvorrichtung nicht versteifbar.

Im Innersten der Schlauchvorrichtung ist ein Innenraum, der von dem Innenschlauch umgeben ist, sodass im Innenraum ein Lumen entsteht, was im ordnungsgemässen Einsatz nicht durch Knicke oder Druck auf die Schlauchvorrichtung verkleinert wird. Dieses Lumen weist zu den Enden der Schlauchvorrichtung jeweils eine Öffnung auf.

Dazu ist es wichtig, dass der Innen- und Aussenschlauch radial steif ausgebildet sind. Diese radiale Steifheit bedingt, dass es zu keinen oder nur kleinen Ausdehnungen und Verengungen des Innen- und Aussenschlauchs kommt. Somit ist sichergestellt, dass es zu keinen Knicken der Schläuche kommen kann, die den Innenraum stark verengen und die Einführung und Benutzung von Vorrichtung in der Schlauchvorrichtung einschränken würden.

Dennoch ist eine hohe Flexibilität und Verbiegbarkeit der Schlauchvorrichtung möglich, damit die Schlauchvorrichtung Windungen entlang der Gefässe und Verzweigungen der Gefässe vollziehen kann.

Die radiale Beweglichkeit der Versteifungsschicht hingegen ist wichtig, damit der Versteifungsabschnitt durch Druck versteift werden kann, da dies durch Formschluss und/oder Kraftschluss nur möglich ist, wenn die Versteifungsschicht durch den aufgebauten Druck von innen gegen den Aussenschlauch gedrückt werden kann.

In der Schlauchvorrichtung kann vorzugsweise zwischen Innenschlauch und Versteifungsschicht ein erweiterbarer, insbesondere elastischer und/oder auffaltbarer, Druckschlauch angeordnet sein.

Der Druckschlauch kann Druck ausgesetzt werden und somit die Versteifungsschicht gegen den Aussenschlauch drücken und den Versteifungsabschnitt der Schlauchvorrichtung versteifen. Diese Anordnung bietet den Vorteil, dass der Druckschlauch zudem einen Sicherheitsmechanismus darstellt.

Es ist anzumerken, dass diese Anordnung vorteilhaft für potentiell auftretende Lecks im Schlauch ist. Die Sicherheit des Patienten wäre trotz eines Lecks gewährleistet, da der Druck zwischen dem Innenschlauch und der Versteifungsschicht im Druckschlauch aufgebaut wird. Es müsste also ein doppeltes Leck auftreten, damit der Druck über den Aussenschlauch entweichen könnte.

Ausserdem kann somit ein Druck auf die Versteifungsschicht ausgeübt werden, ohne dass die Versteifungsschicht eine dichte Schicht aufweisen müsste. Es ist auch eine Versteifungsschicht aus spiralförmig angeordneten Elementen vorstellbar oder eine Versteifungsschicht, die einen Verschluss mit einer komplementär ausgebildeten Innenoberfläche des Aussenschlauchs zum Versteifen der Schlauchvorrichtung bildet. Die Versteifungsschicht ist vorzugsweise dennoch nicht monolithisch gefertigt. Diese bevorzugte Gestaltung der Schlauchvorrichtung ermöglicht eine flexiblere Ausgestaltung der Versteifungsschicht, da sie eine nicht geschlossene, beispielsweise perforierte, gerillte, spiralförmige, maschenförmige, verflochtene oder netzförmige Struktur, Konstruktion oder Oberfläche, aufweisen kann. Dies kann sich vorteilhaft auf die Versteifung des Versteifungsabschnitts auswirken, da der Versteifungsabschnitt somit auch leichter während starker Verbiegung versteift werden kann.
Der Druckschlauch kann expandieren, wenn er Druck ausgesetzt wird, und umfasst vorzugsweise elastisch verformbares Material, und/oder einen faltbares Material, beispielsweise einen Kunststoff.
Besonders geeignet für einen auf- und einfaltbaren Druckschlauch sind Nylon, Polyethylenterephthalat und/oder Polyether-Block-Amid.

Insbesondere vorteilhafterweise umfasst der Druckschlauch dehnbare Elastomere oder anderes thermoplastisches Kunststoffmaterial, wie beispielsweise Silikon und/oder thermoplastische Elastomere und/oder faltbare Kunststoffe. Diese Stoffe weisen eine hohe Biokompatibilität auf, wirken nicht toxisch, sind kostengünstig und sind vorzugsweise elastisch sehr gut verformbar.

Besonders vorteilhaft ist ein Druckschlauch, der einen zusammengefalteten Zustand ohne Einwirkung von zusätzlichem Druck aufweisen kann und einen entfalteten Zustand bei Aufbringen eines Druckes aufweisen kann. Durch einen solchen auffaltbaren Druckschlauch ist eine fest vorgegebene Maximal- und Minimalgrösse beim Anlegen von ordnungsgemässen Drücken gewährleistet und es kann eine Versteifung mit maximierter Dichtheit gewährleistet werden.

Die Versteifungsschicht kann zumindest zwei Stabilisierungselemente umfassen, wobei die Ausrichtung der Stabilisierungselemente zumindest einen Teilvektor in Längsrichtung der Schlauchvorrichtung aufweist und die Stabilisierungselemente vorzugsweise gegeneinander verschiebbar sind.

Die einzelnen Stabilisierungselemente können im nicht versteiften Zustand gegeneinander beweglich, insbesondere verschiebbar, sein. Bevorzugt sind die Stabilisierungselemente wenig zugelastisch und vorzugsweise zudem wenig druckelastisch in Längsrichtung.

Die Stabilisierungselemente der Versteifungsschicht sind vorzugsweise in höherem Mass entlang der Längsrichtung ausgebildet, sodass der Winkel der Stabilisierungselemente zwischen Längsachse der Schlauchvorrichtung und Stabilisierungselement in einem Bereich zwischen -45° und 45°, insbesondere zwischen -30° und 30°, ausgebildet ist. Es ist auch möglich verschiedene Ausrichtungen, also zwei oder mehr unterschiedliche Winkel zur Längsachse der Stabilisierungselemente bezüglich der Längsachse vorzusehen. Dies hat den Vorteil, dass die Verbiegbarkeit nur gering eingeschränkt wird, aber der Formschluss während der Versteifung in Längsrichtung zwischen der Versteifungsschicht und dem Aussenschlauch und vorzugsweise dem Druckschlauch verstärkt wird.

Die Stabilisierungselemente sind vorzugsweise so geformt, dass sie durch hohen Druck in Richtung des Aussenschlauchs eine hohe Reibung mit dem Aussenschlauch aufweisen und zu einer Versteifung der Schlauchvorrichtung führen können.

Die Versteifungsschicht und/oder Stabilisierungselemente umfassen vorzugsweise Metalle und/oder Kunststoffe. Insbesondere geeignet sind beispielsweise Edelstahl oder Polyamid.

Dieser Zusammenhalt zwischen den einzelnen Stabilisierungselementen der Versteifungsschicht kann durch mechanische Adhäsion, wie formschlüssige Verklammerung in Vertiefungen, und/oder durch spezifische Adhäsion ausgebildet werden.

In diesem Zusammenhang wäre auch die Verwendung von Stabilisierungselementen auf dem Aussenschlauch oder Verwendung von zusätzlichen Stabilisierungselementen auf der Innenseite des Aussenschlauchs vorstellbar, die zum Versteifen zum Beispiel durch Formschluss beitragen könnten.

Die Stabilisierungselemente der Schlauchvorrichtung bestehen vorzugsweise aus Metall und/oder Kunststoff.
Insbesondere ist vorzugsweise eine Vielzahl von Stabilisierungselementen zu einem Geflecht verflochten.

Metalle und Kunststoffe haben sehr gute elastische Eigenschaften und sind meistens kostengünstig. Ausserdem ist beim Material eine gute Elastizität sehr wichtig, sodass beim Wegfallen der einwirkenden Kraft durch den Druck in die Ursprungsform zurückgekehrt werden kann. Somit wird gewährleistet, dass die Schlauchvorrichtung versteift werden kann, aber auch wieder elastisch verformbar werden kann, wenn der Druck reduziert wird. Vorteilhaft ist ebenfalls, wenn keine starke elastische Hysterese des Materials vorliegt, also nach dem Entfernen der auslenkenden Kräfte eine Deformation zurück bleiben kann. Einer elastischen Hysterese wird durch die Struktur eines Geflechts, Verwendung eines elastischen Materials und/oder einer nicht plastischen Verformung entgegen gewirkt.

Die Elastizität der Versteifungsschicht kann anstatt oder zusätzlich zur Materialelastizität auch durch die Art der Versteifungsschicht erzielt werden. Ein relativ lockeres Geflecht kann durch Aufbringen eines Druckes eine gewisse elastische radiale Verformung erzielen auch wenn das Material des Geflechtes an sich nicht oder kaum elastisch ist.

Die Verwendung von Stabilisierungselementen aus Metall und/oder Kunststoff erlaubt zudem eine kostengünstige Herstellung. Ein Geflecht kann maschinell sehr schnell gefertigt werden und verringert somit die Kosten der Erfindung.

Eine besonders vorteilhafte Gestaltung ergibt sich für die oben beschriebenen eher spitzen Winkel, in Kombination mit einem Geflecht.

Dennoch wären auch andere Herstellungsmöglichkeiten zum Erzeugen einer vorteilhaften Struktur der Stabilisierungselemente, wie Laserschneiden vorstellbar.

Der Innenschlauch und der Aussenschlauch der Schlauchvorrichtung sind vorzugsweise druckdicht ausgebildet und insbesondere aus einem Kunststoff und/oder Metall hergestellt.

Der Innen- und Aussenschlauch weisen vorzugsweise beide eine Wanddicke von in einem Bereich von 0,01 bis 0,8 mm, insbesondere von 0,2 mm, auf und können Materialien wie Edelstahl, Platin, Iridium, Barium, Wolfram und/oder Plastik, insbesondere Thermoplasten wie Silikone, Polyetheretherketon, Fluorpolymere, Polyvinylchlorid und Polyurethan umfassen.

Bei der Wahl des Materials muss darauf geachtet werden, dass sowohl die mechanischen Eigenschaften, in Bezug auf die Flexibilität, die Oberflächenstruktur, in Bezug auf die Thrombogenität, und die Chemie der Oberfläche, in Bezug auf Beschichtbarkeit und Ladung, vorteilhaft sind. Diese vorteilhaften Eigenschaften weisen die obig genannten Materialien auf und eignen sich somit für die Verwendung in der Schlauchvorrichtung.

Vorzugsweise sind der Innen- und Aussenschlauch zudem armiert, sodass sich die Schläuche nicht axial verdrehen, der Querschnitt unter Druck nicht so stark vergrössert und/oder komprimiert wird und der Schlauch höhere Druckbeständigkeit aufweist. Für die Armierung kann der Innen- und Aussenschlauch durch Spiralen, Maschen, einen Draht oder ein Geflecht mit Querorientierung stabilisiert werden. Die Armierungsstruktur ist vorzugsweise auf ein Verhältnis des Widerstandes zu der radialen Ausdehnung und longitudinalen Ausdehnung optimiert, indem zum Beispiel die Dicke der Armierung und der Winkel zur Schlauchvorrichtung angepasst wird.

Die Wahl dieser Stoffe und einer vorteilhaften Armierung für den Innenschlauch und Aussenschlauch gewährleisten, dass die Schlauchvorrichtung elastisch verformbar ist, aber bei sachgemässer Benutzung nicht zusammengedrückt werden kann. Somit ist immer gewährleistet, dass der Innenraum oder die Lumina frei sind, aber die Schlauchvorrichtung sich dem Verlauf der Gefässe elastisch anpassen kann. In Längsrichtung sollten Aussen- und Innenschlauch zudem vorzugsweise nicht oder kaum elastisch verformbar sein.

Die Schlauchvorrichtung umfasst vorzugsweise eine Fluidkupplung, durch die ein Fluid in den Bereich zwischen Innenschlauch und Versteifungsschicht, insbesondere in den Druckschlauch, einbringbar ist.

Die Fluidkupplung der Schlauchvorrichtung ist vorteilhaft, da somit Fluid in den Bereich zwischen dem Innenschlauch und der Versteifungsschicht eingebracht werden kann. Das Fluid kann eine Flüssigkeit sein, beispielsweise Wasser, Salzwasser oder auch ein Gas. Somit erleichtert das Einbringen eines Fluids zwischen den Innenschlauch und die Versteifungsschicht, insbesondere in den Druckschlauch, das Versteifen der Schlauchvorrichtung. Bevorzugt ist jedoch die Verwendung einer Flüssigkeit, da dies beim Entweichen weniger gefährlich für Menschen ist, als ein Gas, insbesondere Luft.

Die Schlauchvorrichtung kann vorzugsweise zumindest teilweise ein im Röntgen sichtbares Material, insbesondere Metall, umfassen.

Bei minimal-invasiven Operationen mit Schlauchvorrichtungen kann die Position der Schlauchvorrichtung und insbesondere des eingeführten Endes der Schlauchvorrichtung mit dem blossen Auge nicht einsehbar sein. Demnach muss vorzugsweise ein bildgebendes Verfahren verwendet werden, um die eingeführte Schlauchvorrichtung sichtbar zu machen. Vorzugsweise wird ein Röntgenverfahren, wie die Fluoroskopie angewendet, damit vorzugsweise Echtzeitbildgebung der eingeführten Schlauchvorrichtung gewährleistet werden kann.

Da die Abschwächung von Röntgenstrahlen durch den Massenschwächungskoeffizient gegeben ist, sollte ein Material mit Elementen einer hohen Ordnungszahl gewählt werden.

Da Plastik oftmals hauptsächlich aus Kohlenstoffverbindungen besteht, die Röntgenstrahlung schlecht absorbieren, sollte die Schlauchvorrichtung stattdessen sichtbares Material wie modifiziertes Plastik oder Metalle enthalten. Bereits kleine Mengen von beispielsweise Eisen können die Sichtbarkeit der Schlauchvorrichtung gewährleisten.

Ein vorderer Bereich der Schlauchvorrichtung kann vorzugsweise konisch ausgebildet sein, damit keine Stufe entsteht.

Der vordere Bereich der Schlauchvorrichtung ist gemäss der Erfindung der Teil, der als erstes in das Gefäss des Patienten eingeführt wird.

Der vordere Bereich der Schlauchvorrichtung sollte möglichst konisch ausgebildet sein, da dies den Vorteil bietet, dass die Verletzungsgefahr bei Verwendung der Schlauchvorrichtung minimiert wird. Beim Einführen der Schlauchvorrichtung in einen Patienten verursacht ein konischer vorderer Bereich der Schlauchvorrichtung geringere oder keine Verletzungen und Schmerzen. Zudem ist die benötigte Kraft geringer und die Anwendung leichter durchzuführen. Auch beim Führen entlang der Gefässe eines Menschen ist ein konischer vorderer Bereich von Vorteil, da zum einen die Kurven in den Gefässen leichter zu vollführen sind, wenn es sich um einen schmaleren vorderen Bereich handelt, der zudem somit leichter elastisch verformbar ist. Zum anderen minimiert ein konischer vorderer Bereich eine Verletzung von Gefässen und das potentielle Lösen von Ablagerungen oder Gerinnseln.

Aussen auf dem Aussenschlauch der Schlauchvorrichtung kann eine hydrophile oder hydrophobe Beschichtung angeordnet sein. Zudem ist eine Beschichtung im Innenschlauch mit Polytetrafluoroethylene besonders vorteilhaft.

Die Beschichtung des Aussenschlauchs kann unter anderem das Gleitverhalten verbessern und die Reibung minimieren. Auf der anderen Seite muss Biokompatibilität gewährleistet sein oder die Infektionsgefahr gering gehalten wird, sowie Hämokompatibilität, Antithrombogenität oder geringe Partikelfreisetzung optimiert wird. Dazu eignen sich besonders gut Beschichtungen durch Polymere.

In den Beschichtungsverfahren wird die Oberfläche vorzugsweise vorher aktiviert, um Beschichtungen aufnehmen zu können. Dies kann beispielsweise durch die Plasmaaktivierung erfolgen, bei der ein Elektronenplasma dazu führt, dass offene Bindungsstellen auf der Oberfläche entstehen, die mit polaren Hydroxy-Gruppen besetzt werden. Somit entsteht eine hydrophile Oberfläche und die Polymerbeschichtung kann besser haften.

Die zu beschichtende Oberfläche kann beispielsweise durch Plasmapolymerisation im Vakuum durch Zuführen von Gas mit Precursor-Monomeren, die sich auf der Oberfläche ablagern, je nach Precursor-Monomeren, hydrophil oder hydrophob gemacht werden. Andere Beschichtungsverfahren wie Tauchbeschichtung, Spritzbeschichtung, Reel-to-Reel-Beschichtung sind ebenfalls vorstellbar.

In einem letzten Schritt können die Moleküle der Beschichtung noch stärker vernetzt werden, indem thermische Energie oder UV-Strahlung zugeführt wird.

Als hydrophobe Beschichtung für den Aussenschlauch eignet sich Polytetrafluorethylen, da das Molekül durch den symmetrischen Aufbau unpolar ist, eine sehr geringe Oberflächenspannung aufweist und geringe Kohäsions- und Adhäsionskräfte zu anderen Substanzen aufweist. Weiterhin wären auch anderes Polyfluorethylen, Hexafluorpropen und/oder Polydimethylsiloxan als hydrophobe Beschichtungen vorstellbar, wobei Polydimethylsiloxan sogar vorteilhafter Weise antithrombogen wirkt.

Als hydrophile Polymere eignen sich hingegen Beschichtungen, die Wasserstoffbrückenbindungen ausbilden können, sodass ein Wasserfilm leicht an die Oberfläche gebunden werden kann. Hydrophile Beschichtungen können nur aus einem hydrophilen Polymer bestehen, oder eine Kombination von hydrophilen Polymeren umfassen. Vorteilhafte vorstellbare hydrophile Beschichtungen sind unter anderem Hydrogele oder auch Polyvinylpyrrolidon, welches in seinen Eigenschaften durch Zugabe von Vinylpyrrolidon-Copolymere weiter angepasst werden kann.

Der Versteifungsabschnitt der Schlauchvorrichtung kann einen Teil oder die gesamte Schlauchvorrichtung bilden.

Die Länge und der Bereich des Versteifungsabschnitt kann vorteilhafterweise leicht auf die jeweilige Problemstellungen angepasst werden. Besonders vorteilhaft ist eine Länge der Versteifungsschicht von rund 30 cm bis an das vordere Ende, oder eine komplette Versteifbarkeit der Schlauchvorrichtung.

Somit kann der Versteifungsabschnitt nur den Bereich einnehmen, der benötigt wird, um die Schlauchvorrichtung in einem Bereich zu fixieren. Der Rest der Schlauchvorrichtung kann von den Vorteilen einer fehlenden Versteifungsschicht profitieren, wie beispielsweise erhöhter Elastizität. Somit können zudem Kosten reduziert werden und dennoch eine vollständige Funktionsweise gewährleistet werden.

Die gesamte Länge der Schlauchvorrichtung ist vorzugsweise je nach gewünschtem Verwendungszweck zwischen 10 und 120 cm lang und die Länge des Versteifungsabschnitts kann sich über die gesamte Länge, oder einen Abschnitt erstrecken.

Der Innenschlauch der Schlauchvorrichtung kann einen Innendurchmesser von 1 bis 11,33 mm aufweisen.

Ein kleiner Aussendurchmesser ist für minimal-invasive Eingriffe sehr wichtig. Nichtsdestotrotz ist ein gewisser minimaler Innendurchmesser für die Durchführung von Drähten, Werkzeugen und/oder Stents notwendig. Der Innendurchmesser von Kathetern wird in French angegeben, wobei ein French 0.33 mm entspricht. Somit ist weist die Schlauchvorrichtung einen Innendurchmesser von 3-32 Fr auf. Damit ein kleiner Innendurchmesser erreicht werden kann, wodurch Werkzeug und beispielsweise Stents eingeführt werden können, und die Versteifbarkeit der Schlauchvorrichtung gewährleistet ist, müssen die Schlauchvorrichtungsschichten auch sehr dünnwandig produziert werden. Ein kleiner Innendurchmesser ist notwendig, um in engen Gefässen Platz zu finden, oder Windungen von bis zu rund 180° mit der Schlauchvorrichtung in Gefässen vollziehen zu können. Deshalb ist es ein besonders vorteilhaftes Merkmal der Erfindung, dass die Schlauchvorrichtung selbst bei geringem Innendurchmesser voll funktionsfähig ist und mit sehr geringen Kosten hergestellt werden kann. Der Aussendurchmesser ist vorzugsweise maximal 4 Fr, insbesondere maximal 2 Fr, grösser als der Innendurchmesser. Somit ist bei einem Innendurchmesser von 6 Fr der Aussendurchmesser höchstens 10 Fr gross, sodass die Schlauchvorrichtung sehr kompakt ausgebildet ist und leichter um Verzweigungen von Gefässen geführt werden kann.

Der Innenschlauch der Schlauchvorrichtung kann vorzugsweise mindestens zwei Lumina aufweisen, sodass mehrere Vorrichtungen in getrennten Lumina durch die Schlauchvorrichtung eingeführt werden können.

Mehrere getrennte Lumina in einer Schlauchvorrichtung können von Vorteil sein, damit sich bei gleichzeitiger Verwendung von Werkzeugen und/oder Geräten nicht so leicht Verhakungen oder Verklemmungen stattfinden können. Dies bietet den Vorteil, dass mehrere Funktionen, wie beispielsweise das Führen des Führungsdrahts und Einführen, und/oder Vorbereiten von Werkzeug und/oder Geräten gleichzeitig durchgeführt werden kann. Ausserdem kann somit ein Formdraht parallel zu einem Führungsdraht in jeweils einem Lumen verwendet werden. Vorzugsweise wird dennoch ein zusätzlicher weicher Dilatator verwendet.

Das Set umfasst mindestens eine Schlauchvorrichtung wie vorhergehend beschrieben und einen Dilatator, insbesondere mindestens einen weichen Dilatator und einen Formdraht und/oder Führungsdraht.
Somit ist der volle Funktionsumfang gewährleistet und alle Komponenten sind kompatibel.

Der Draht kann ein Führungsdraht und/oder ein Formdraht sein.

Der Führungsdraht und der Formdraht sind in den Innenschlauch einführbar. Insbesondere weist der Formdraht Biegungen für die Führung der Schlauchvorrichtung auf und/oder ist zu Biegungen verformbar ausgebildet. Vorzugsweise kann eine Biegung des Formdrahtes angepasst werden, sodass der Formdraht flexibel verwendet werden kann, wie aus dem Stand der Technik bekannt.

Ein Formdraht ist insbesondere rotationsstabil und/oder torsionsstabil um die Dilatatorspitze zu führen.

Der Formdraht und/oder das Lumen, in das er einführbar ist, sind vorzugsweise beschichtet, behandelt oder aus einem sehr reibungsarmen Material geformt, damit der Formdraht möglichst leicht ein- und ausgeführt werden kann.

Übliche Formen für Formdrähte sind gerade Drähte, Hockey-Stick, Kobra, SOS und Berenstein. Der Formdraht kann ein oder zwei geformte Enden aufweisen.

Zudem umfasst das Set vorzugsweise mindestens einen Dilatator. Insbesondere ist der Dilatator leicht verformbar ausgebildet, und somit ein weicher Dilatator.

Der Formdraht dient dazu, dass der flexible vordere Bereich der Schlauchvorrichtung und/oder der Dilatator als Formvorrichtung in den Gefässen gesteuert werden kann. Dazu ist der Formdraht vorzugsweise in Abhängigkeit des Verwendungszwecks gebogen, und in die Schlauchvorrichtung einführbar. Die Biegung des Formdrahtes ist vorzugsweise sehr flexibel, sodass beim Einführen erst am Ende der Schlauchvorrichtung und/oder am Dilatator eine Biegung durch den Formdraht vorgegeben wird, da die Steifigkeit des Formdrahtes nur am Ende der Schlauchvorrichtung und/oder am Dilatator ausreicht um diese zu verbiegen. Vorzugsweise ist die Schlauchvorrichtung nicht durch den Formdraht verbiegbar, sondern nur der weiche Dilatator. Die Biegung ist somit durch den Formdraht vollziehbar und die Schlauchvorrichtung kann anschliessend weiter in das gewünschte Gefäss eingeführt oder versteift werden.

Der weiche Dilatator ist bevorzugt länger als die jeweilige Schlauchvorrichtung ausgebildet, insbesondere mindestens 15 cm länger.

Der weiche Dilatator kann über seine gesamte Länge, oder nur an der Spitze des weichen Dilatators weich ausgebildet sein.

Wenn der weiche Dilatator mit horizontaler Längsachse fixiert wird, sodass die vordersten 5 cm zur verjüngten Spitze des Dilatators frei sind, und ein Gewicht von 30 g an der Spitze des Dilatators orthogonal Kraft ausübt, lenkt sich die Spitze des weichen Dilatators um einen Bereich von 30 mm bis 48 mm, insbesondere 42 mm, aus. Im Vergleich dazu lenkt sich die Spitze eines harten Dilatators nur um 23 mm aus. Die Auslenkung eines weichen Dilatators ist also im Vergleich zu einem harten Dilatator im Wesentlichen mindestens 25% grösser bei gleicher Formgebung der Dilatatoren.
Die Verjüngung des weichen Dilatators ist von der Spitze, an der Öffnung für den Führungsdraht, bis zur Aufweitung auf den Innendurchmesser der Schlauchvorrichtung 20 bis 80 mm, insbesondere 40 mm, lang.

Der weiche Dilatator kann zwei Lumina aufweisen, ein Lumen für den Führungsdraht und ein Lumen für den Formdraht. Das Lumen für den Formdraht ist in der Spitze des Dilatators abgeschlossen, das Lumen für den Führungsdraht ist offen.

Ein abgeschlossenes Lumen für den Formdraht hat den Vorteil, dass der Formdraht nur zum Ausbilden der Form verwendet werden und die Gefässe nicht verletzen kann. Zudem ist der Formdraht vorzugsweise nicht spitz zulaufend sondern abgeflacht ausgebildet, sodass der Innenraum des Lumens, sowie das abgeschlossene Ende nicht beschädigt werden kann.

Ein weiteres Lumen bietet zudem den Vorteil, dass parallel zur Formbarkeit des Dilatators und/oder der vorderen Schlauchvorrichtung durch den Formdraht ein Lumen zum Einführen von Substanzen verwendet werden kann. Somit kann beispielsweise ein Kontrastmittel in das Gefäss eingebracht werden, ohne den Formdraht entfernen zu müssen.

Der Innendurchmesser eines Lumens des Dilatators in Bezug auf die Anwendung eines Führungsdrahtes liegt vorzugsweise in einem Bereich von im Wesentlichen 0.2 mm bis 1.2 mm, insbesondere 0,35 mm bis 0,97 mm.

Der Übergang zwischen der Schlauchvorrichtung und dem Dilatator schliesst vorzugsweise im Wesentlichen formschlüssig ab und der Dilatator weist in diesem Bereich vorzugsweise einen Aussendurchmesser von im Wesentlichen 1 mm bis 11,33 mm auf.

Der weiche Dilatator kann hydrophil beschichtet sein, ebenfalls durch die bereits vorgängig bezüglich der Schlauchvorrichtung dargelegten Massnahmen. Der weiche Dilatator ist bevorzugt weicher als der Formdraht, so dass der Dilatator durch den Formdraht verformt werden kann.

Bevorzugt ist der weiche Dilatator im Röntgen sichtbar, insbesondere durch die bereits bezüglich der Schlauchvorrichtung dargelegten Massnahmen.
Insbesondere sind mindestens die vordersten 4 cm des Dilatators durch Röntgenverfahren zu lokalisieren.

Der weiche Dilatator kann auch unabhängig der vorgängig beschriebenen Schlauchvorrichtung mit bisherigen Schlauchvorrichtungen verwendet werden.

Der weiche Dilatator kann aus Silikon oder thermoplastischen Kunststoffen hergestellt sein.
Der weiche Dilatator kann durch farbliche Markierung von herkömmlichen harten Dilatatoren, oder im Set enthaltenen Dilatatoren kenntlich gemacht sein.

Der harte Dilatator wird als erstes Teil vor der Schlauchvorrichtung nach Einführen des Führungsdrahtes in das Gefäss eingeführt und weitet das Gefäss temporär für das Einführen der Schlauchvorrichtung. Dazu ist der Dilatator konisch geformt.

Das Set kann zusätzlich einen harten Dilatator umfassen. Somit kann der Arzt wählen, welcher Dilatator in der Situation besser passt.

Zur Lösung der Aufgabe führt weiterhin ein Verfahren zur Behandlung eines Patienten, wobei eine Schlauchvorrichtung wie vorhergehend beschrieben in ein Gefäss eines Patienten eingeführt wird.

Ein derartiges Verfahren führt zu einer sicheren und schnellen Behandlung des Patienten.

Die Schlauchvorrichtung kann versteift werden, sobald die Schlauchvorrichtung an einem Zielgefäss positioniert ist.

So wird ein sicherer Zugang für die eigentliche Behandlung des Zielgefässes ermöglicht.

Wie auch bisher üblich, wird bevorzugt zunächst mit einer Nadel ein Zugang in ein Zugangsgefäss geschaffen. Durch diese Nadel wird ein Führungsdraht in das Zugangsgefäss eingeführt und die Nadel dann wieder aus dem Gefäss entfernt. Über den Zugangsdraht wird ein Dilatator, insbesondere ein harter Dilatator, in das Zugangsgefäss eingeführt. Der harte Dilatator wird somit vorzugsweise zusammen mit einer Schlauchvorrichtung verwendet und verkauft und ist nach dem Stand der Technik bekannt. Ein harter Dilatator ist so in die Schlauchvorrichtung einführbar, dass er den Führungsdraht umgibt und den Bereich zwischen Führungsdraht und Schlauchvorrichtung einnimmt. Der harte Dilatator ist sehr steif und kann somit zum Aufdehnen der Gefässwand beim Eintritt ins Gefässsystem verwendet werden.

Wenn der harte Dilatator mit horizontaler Längsachse fixiert wird, sodass die vordersten 5 cm zur verjüngten Spitze des Dilatators frei sind, und ein Gewicht von 30 g an der Spitze des Dilatators orthogonal Kraft ausübt, lenkt sich die Spitze des harten Dilatators um einen Bereich von 10 bis 35 mm, insbesondere 23 mm, aus.
Die Verjüngung des harten Dilatators ist von der Spitze an der Öffnung für den Führungsdraht bis zur Aufweitung auf den Innendurchmesser der Schlauchvorrichtung 20 bis 80 mm, insbesondere 40 mm, lang.

Der harte Dilatator kann, bevorzugt anschliessend, gegen einen weichen Dilatator ausgetauscht werden. Der weiche Dilatator kann dann bis vor das Zielgefäss vorgeschoben werden. Der Führungsdraht kann dann in das Zielgefäss vorgeschoben werden. Der Führungsdraht und der Formdraht werden dann fixiert und der Dilatator mit Schlauchvorrichtung ins Zielgefäss vorgeschoben und fixiert. Daraufhin wird die Schlauchvorrichtung ebenfalls in flexiblem Zustand ins Zielgefäss vorgeschoben und versteift.

Nach Versteifen der Schlauchvorrichtung wird der weiche Dilatator mit dem Formdraht herausgezogen.

Der gewünschte Eingriff im Zielgefäss kann daraufhin beginnen.

Somit ergibt sich der Vorteil, dass ein weicher Dilatator gleichzeitig als optimierte Führungsvorrichtung und diagnostisch verwendbar ist. Somit kann zusätzlich Zeit eingespart werden und der Eingriff technisch leichter ohne Materialwechsel vollzogen werden.

Die Aufgabe wird weiter durch ein Verfahren zum selektiven Versteifen einer Schlauchvorrichtung gelöst.

Durch Einbringen eines Fluids in den Zwischenraum zwischen Innenschlauch und Versteifungsschicht wird die Schlauchvorrichtung versteift.

Das selektive Versteifen der Schlauchvorrichtung ermöglicht zum einen die benötigte Flexibilität zum Einführen in die Gefässe und zum anderen die selektive Stabilität einer versteiften Schlauchvorrichtung oder eines Versteifungsabschnitts. Auch die Verwendungszeit wird minimiert, da eine Schlauchvorrichtungen nach dem Stand der Technik oftmals von ihrem Verwendungsort, beispielsweise durch Blutfluss, das Manipulieren in der Schlauchvorrichtung, oder Abrutschen der Schlauchvorrichtung, ausgelenkt werden kann.
Ausserdem ist ein Fluid zum einen aus Sicherheitsgründen möglichst so zu wählen, dass selbst für den Fall einer starken Beschädigung der Schlauchvorrichtung keine gesundheitlichen Schäden entstehen können. In diesem Zusammenhang wäre auch eine Vorrichtung zum Überwachen des Drucks durch das eingebrachte Fluid vorstellbar.

Durch Reduktion des Druckes im Zwischenraum zwischen Innenschlauch und Versteifungsschicht kann die Schlauchvorrichtung wieder flexibel werden. Vorzugsweise ist der Schlauch bei einer Reduktion auf im Wesentlichen atmosphärischen Druck und Druck in den Blutgefässen eines Menschen im elastischen Zustand. Besonders wichtig ist dabei, dass dieses Verfahren selektiv aktiviert und deaktiviert werden kann. Somit kann die Schlauchvorrichtung flexibel eingeführt werden. Am Zielort kann die Schlauchvorrichtung für den minimal-invasiven operativen Eingriff, gemäss individueller Anatomie, versteift werden und anschliessend zum Entfernen wieder elastisch gemacht werden, um mögliche gesundheitliche Schäden zu vermeiden. Mögliche Schäden, wie beispielsweise Schlaganfälle, können durch das Lösen von Ablagerungen oder Thromben, sowie eingeführtes Werkzeug und/oder Geräte und/oder Kleinstteile verursacht werden. Durch eine zumindest teilweise versteifte Schlauchvorrichtung werden diese Risiken minimiert und präziseres Arbeiten ermöglicht.

Es ist daher von besonderer Wichtigkeit diese potentiellen Schäden zu vermeiden und ein grosser Vorteil der Erfindung, dass selektives Versteifen der Schlauchvorrichtung nach Bedarf durch das Einbringen eines Fluids in den Zwischenraum zwischen Innenschlauch und Versteifungsschicht versteift werden kann.

Es ist zudem von Vorteil, dass der Druck nur aufgebaut werden muss um die Schlauchvorrichtung zu versteifen und abgebaut werden muss um sie elastisch zu machen, da somit sichergestellt ist, dass die Schlauchvorrichtung auch bei Versagen der Druckvorrichtung wieder lösbar ist. Müsste ein Druck aufgebaut werden, um sie elastisch zu machen, wäre im Falle einer undichten Schlauchvorrichtung eine versteifte Schlauchvorrichtung in den Gefässen eines Menschen. Diese wäre nicht, oder nur sehr schwer, und/oder unter grossen Gefahren für den Menschen entfernbar.

Im Folgenden werden Ausführungsformen der Erfindung detailliert mit Bezugszeichen beschrieben. Hierbei zeigt
- Figur 1:: einen Versteifungsabschnitt der Schlauchvorrichtung,
- Figur 2:: ein Ausführungsbeispiel des Versteifungsabschnitts,
- Figur 3:: ein Ausführungsbeispiel des Versteifungsabschnitts,
- Figur 4:: ein Ausführungsbeispiel des Versteifungsabschnitts,
- Figur 5:: ein Ausführungsbeispiel des Versteifungsabschnitts,
- Figur 6:: eine Struktur der Versteifungsschicht,
- Figur 7:: eine alternative Struktur der Versteifungsschicht,
- Figur 8:: einen Querschnitt des Versteifungsabschnitts der Schlauchvorrichtung,
- Figur 9:: ein Querschnitt des Versteifungsabschnitts mit eingeführtem Dilatator mit einem Lumen,
- Figur 10:: einen Querschnitt des Versteifungsabschnitts mit eingeführtem Dilatator mit zwei Lumina,
- Figur 11:: eine Schlauchvorrichtung mit Versteifungsabschnitt,
- Figur 12:: ein Set einer Schlauchvorrichtung mit Dilatator und zusätzlichem Führungsdraht,
- Figur 13:: ein Dilatator mit einem eingeführten Führungsdraht,
- Figur 14:: ein Dilatator mit einem eingeführten Führungsdraht und Formdraht,
- Figur 15:: ein Set einer Schlauchvorrichtung mit Dilatator und zusätzlichem Führungsdraht mit partiell entfernten Schichten,
- Figur 16:: ein Längsschnitt eines Dilatators in einer Schlauchvorrichtung mit zwei Lumina.

Identische Bezugszeichen in der Abbildung zeigen identische Komponenten.

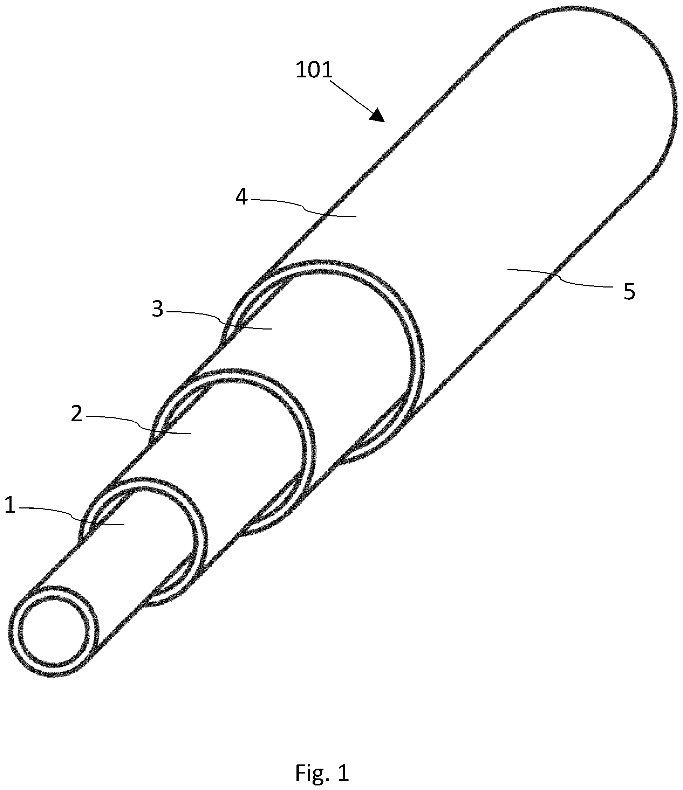

Figur 1 zeigt einen Versteifungsabschnitt 101 der Schlauchvorrichtung 100 einer bevorzugten Ausführungsform der Erfindung mit vier Schlauchschichten. Die Schlauchvorrichtung 100 ist zur besseren Sichtbarkeit in offenen Abschnitten der einzelnen Schichten dargestellt. Von aussen nach innen ist ein Aussenschlauch 4, eine Versteifungsschicht 3, ein Druckschlauch 2 und ein Innenschlauch 1 angeordnet.
Der Druckschlauch 2 wird zum Versteifen mit einer isotonischen Natriumchloridlösung mit einem Druck von 16 bar gefüllt und somit drückt die Versteifungsschicht 3 gegen den Aussenschlauch 4, indem sie radial nach aussen bewegt wird. Beim Entfernen des Drucks wird die Versteifungsschicht 3 zudem wieder radial nach Innen bewegt und die Versteifung des Versteifungsabschnitts 101 lässt nach.

Der Druckschlauch 2 ist in dieser Ausführungsform aus Thermoplasten gefertigt und kann somit durch das Einführen von einer Kochsalzlösung expandiert werden und elastisch radial verformt werden.

Die Versteifungsschicht 3 ist beim Entfernen des angelegten Drucks auch wieder radial nach innen bewegbar. Somit ist sichergestellt, dass die Schlauchvorrichtung 100 immer entfernbar ist, insbesondere wenn der Druck nicht mehr aufgebaut werden kann. Die Schlauchvorrichtung 100 ist im Grundzustand ohne Druck beweglich ausgebildet, sodass keine Gefahr durch eine irreversible Versteifung der Schlauchvorrichtung 100 bei einem Defekt besteht. Die Versteifungsschicht 3 ist in diesem Fall aus einem losen, gegeneinander beweglichen, Geflecht aus Edelstahl und/oder Kunststoff gebildet, welches zu einem Teilvektor in Längsrichtung verläuft. Somit kann die Versteifungsschicht 3 leicht expandieren und es ist eine starke Reibung mit dem Aussenschlauch herstellbar.

Der Aussenschlauch 4 und der Innenschlauch 1 umfassen in diesem Ausführungsbeispiel Polysiloxane und eine Edelstahlspirale, wobei die Edelstahlspirale helikal entlang der Längsachse der Schlauchvorrichtung 100 ausgerichtet ist und im Polysiloxan eingebettet ist und vollständig umschlossen ist. In diesem Zusammenhang wäre aber auch die alternative Verwendung von Polyurethan für den Innen- 1 und Aussenschlauch 4 vorstellbar. Die Innenseite des Innenschlauchs 1 ist mit Polytetrafluorethylen beschichtet, damit ein möglichst geringer Reibungswiderstand innerhalb des Innenschlauchs für eingeführte Geräte, Werkzeuge, Flüssigkeiten und Kleinstteile entsteht. Der Aussenschlauch 4 ist zudem hydrophil durch eine Beschichtung 5 mit Polyvinylpyrrolidon. Somit ist das Einführen in einen Körpergang leichter möglich und atraumatisch durchführbar. Ausserdem werden durch die Beschichtung 5 die Gleiteigenschaften der Schlauchvorrichtung 100 innerhalb der Gefässe erhöht.

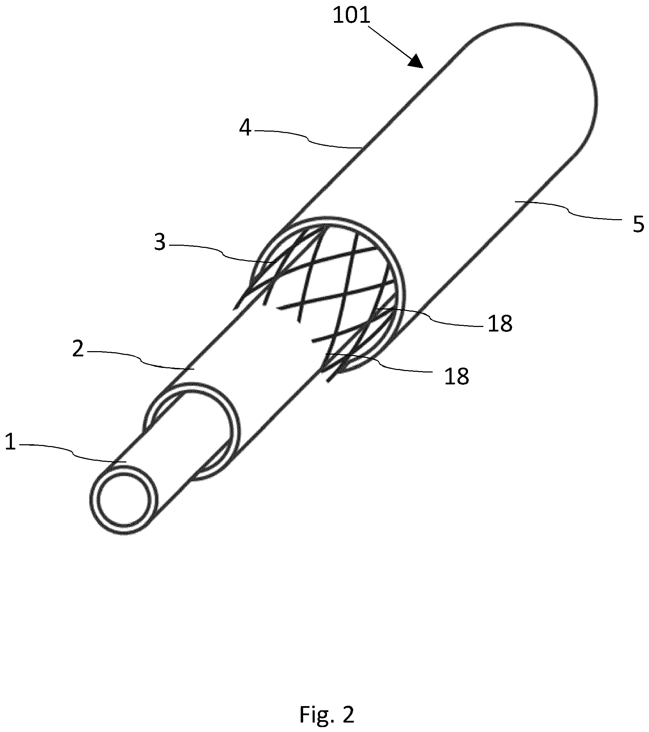

Figuren 2-5 zeigen explizite Ausführungsformen alternativ zu Figur 1. Bei analogen Bezugszeichen, Funktionen und Ausbildungen der Erfindung, die nicht explizit erwähnt werden, wird auf Figur 1 verwiesen.

Figur 2 zeigt den Versteifungsabschnitt 101 der Schlauchvorrichtung 100, die eine Versteifungsschicht 3 mit einem Geflecht aus Edelstahl zeigt, wobei die einzelnen Stabilisierungselemente 18 der Versteifungsschicht 3 gegeneinander verschiebbar und/oder gleitend gefertigt sind. Die Versteifungsschicht 3 ist durch den Druckschlauch 2 ohne starken Widerstand radial nach aussen bewegbar und kann durch Reibung mit dem Aussenschlauch 4 den Versteifungsabschnitt 101 versteifen.
Es sind aber auch andere Materialien für die Stabilisierungselemente 18 wie zum Beispiel Polyamid vorstellbar.

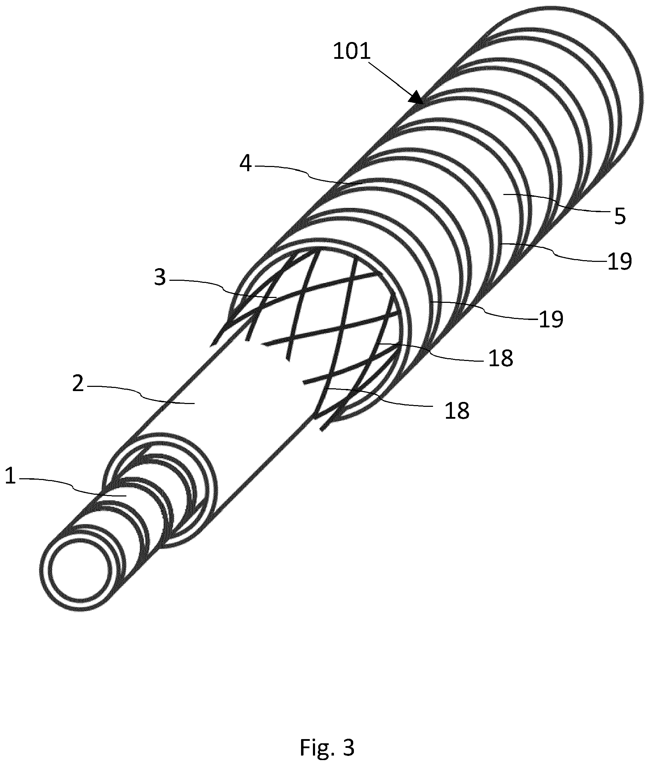

Figur 3 zeigt ein Ausführungsbeispiel des Versteifungsabschnitts 101 der Schlauchvorrichtung 100 analog zu Figur 2. Zusätzlich zu dem Geflecht aus Edelstahl der Versteifungsschicht 3 mit Stabilisierungselementen 18, wurde die Armierung 19 aus Edelstahl des Innen- und Aussenschlauchs 1, 4 dargestellt.
Die Armierung 19 ist dabei möglichst konzentrisch um die Längsachse der Schlauchvorrichtung 100 ausgebildet, sodass die Schlauchvorrichtung 100 weiterhin möglichst flexibel zu den Seiten ausgebildet ist, wenn sie sich nicht im versteiften Zustand befindet. Die Armierung 19 ist in diesem Ausführungsbeispiel durch Ringe realisiert, aber auch eine spiralförmige Armierung 19 und eine maschenförmige Armierung 19 sind vorstellbar.

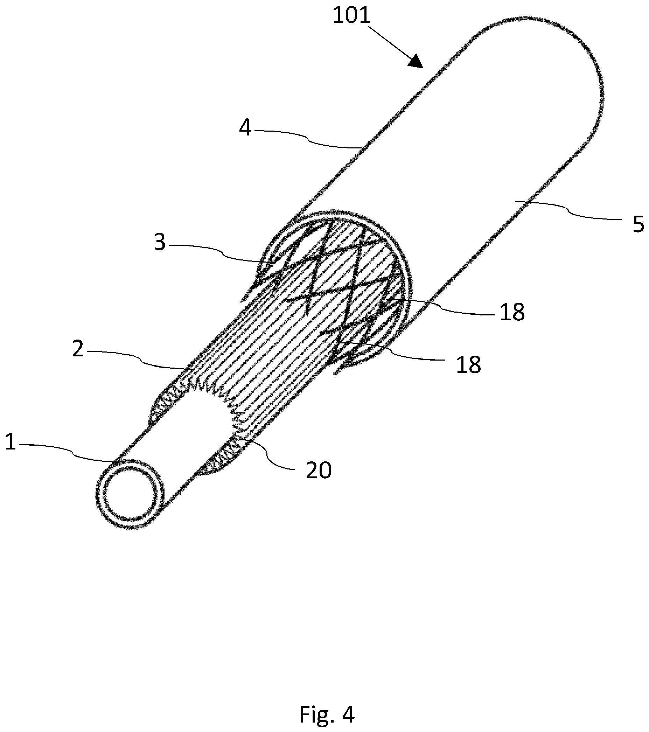

Figur 4 zeigt ein Ausführungsbeispiel des Versteifungsabschnitts 101 der Schlauchvorrichtung 100, wobei der Druckschlauch 2 durch Entfaltung einer gefalteten Struktur 20, die Versteifungsschicht 3 und Stabilisierungselemente 18 radial nach aussen gegen den Aussenschlauch 4 drücken kann. Somit verändert sich die Ausdehnung des Druckschlauches 2 auch nach mehrmaliger Verwendung nicht, da keine oder im Wesentlichen keine elastische Verformung stattfindet. Die gefaltete Struktur 20 des Druckschlauchs 2 faltet sich auf, sobald mit dem Druckschlauch 2 ein Druck durch das Einführen der Salzlösung angelegt wird und faltet sich wieder ein, wenn der Druck entfernt wird. Dies hat zudem den Vorteil, dass die elastische Hysterese durch die gefaltete Struktur 20 minimiert wird. Es wird also minimiert, dass das Verbleiben einer Deformation nach dem Entfernen der auslenkenden Kraft fortbesteht. Somit wird die Sicherheit, sowie die Haltbarkeit des Druckschlauchs 2 der Schlauchvorrichtung 100 gewährleistet.

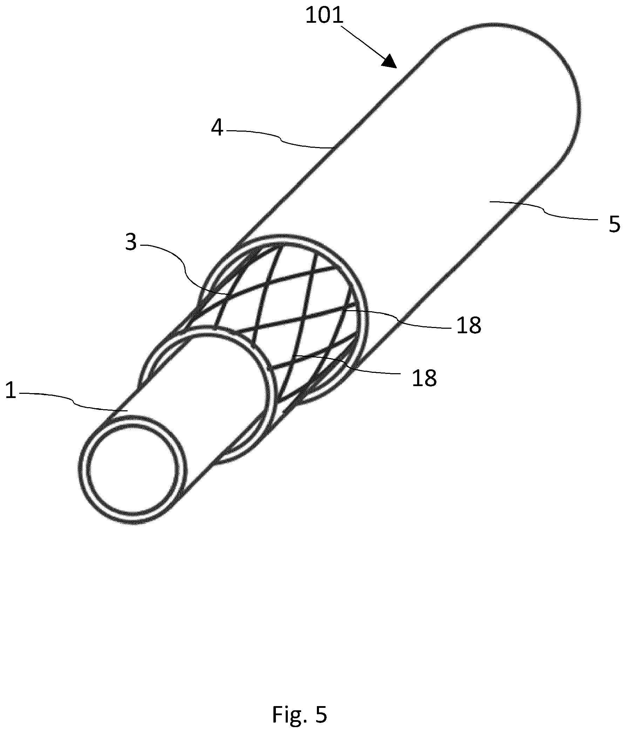

Figur 5 zeigt ein Ausführungsbeispiel des Versteifungsabschnitts 101 der Schlauchvorrichtung 100, wobei die Versteifungsschicht 3 einen dichten Schlauch als eine Einheit mit einem Kunststoff und den Stabilisierungselementen 18 ausbildet. Die Versteifungsschicht 3 kann somit zusätzlich die Funktion des Druckschlauches 2 übernehmen und durch einen angelegten Druck radial nach aussen gegen den Aussenschlauch 4 gedrückt werden, um den Versteifungsabschnitt 101 zu versteifen.

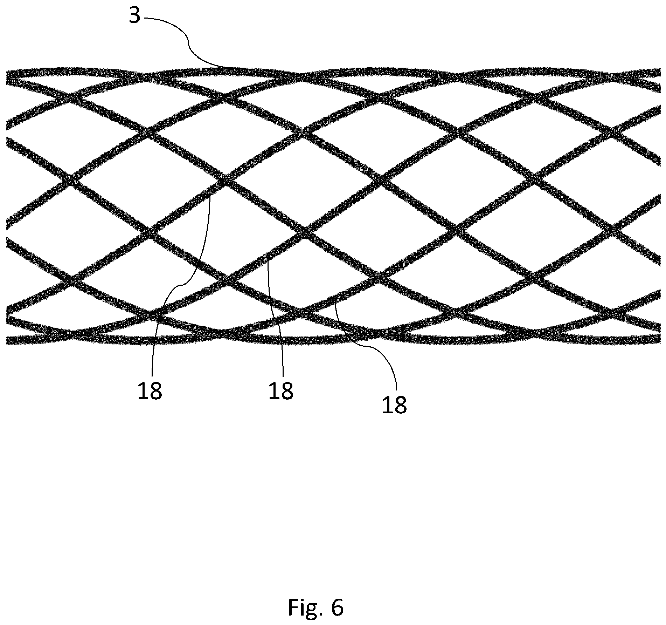

Figur 6 zeigt ein Ausführungsbeispiel der Versteifungsschicht 3 mit Stabilisierungselementen 18, wobei das gebildete Geflecht an den Schnittstellen nicht starr miteinander verbunden ist, sondern gegeneinander frei beweglich ausgebildet ist, um radiale Verformbarkeit zu gewährleisten. Die Stabilisierungselemente 18 sind in diesem Ausführungsbeispiel in einem Winkel von im Wesentlichen 30° zur Längsachse der Schlauchvorrichtung 100 ausgebildet.

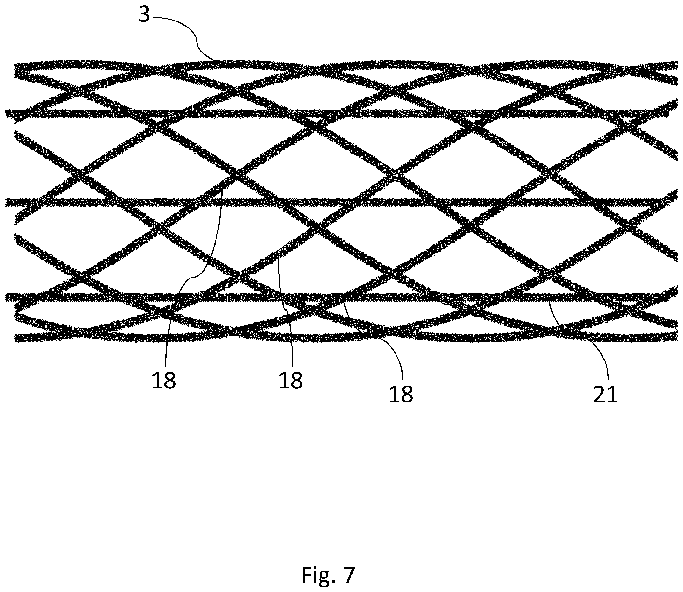

Figur 7 zeigt ein weiteres Ausführungsbeispiel der Versteifungsschicht 3 mit Stabilisierungselementen 18 analog zu Figur 6, wobei zusätzlich longitudinale Stabilisierungselemente 21 im Wesentlichen parallel zur Längsachse dargestellt sind, die die Reibung zusätzlich verstärken, wenn sie radial gegen den Aussenschlauch 4 gedrückt werden.

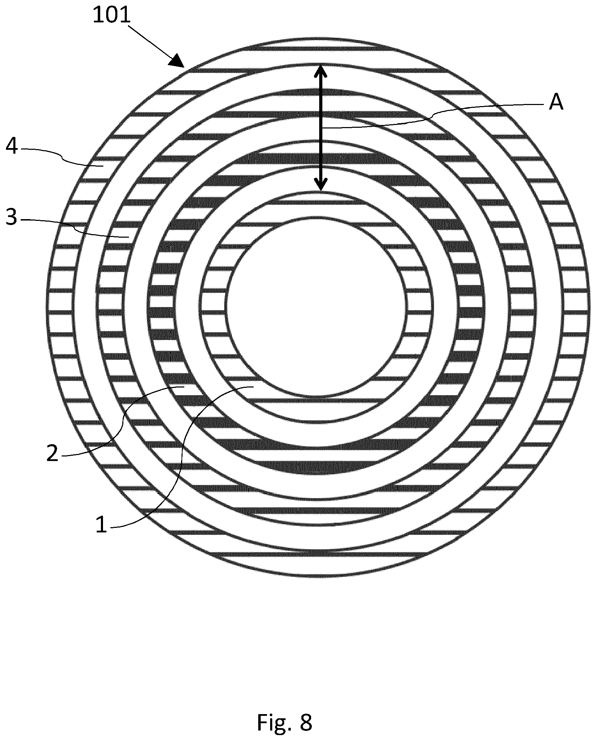

Figur 8 zeigt einen Querschnitt des Versteifungsabschnitts 101 der Schlauchvorrichtung 100 mit vier Schlauchschichten. Von aussen nach innen ist ein Aussenschlauch 4, eine Versteifungsschicht 3, ein Druckschlauch 2 und ein Innenschlauch 1 zu sehen. Zudem wurde der Bereich A zwischen dem Innenschlauch und dem Aussenschlauch 4, zwischen denen der Druck aufgebaut werden kann, markiert. Durch den angelegten Druck zwischen Innenschlauch 1 und Druckschlauch 2 ist eine radiale Bewegung der Versteifungsschicht 3 durch den Druckschlauch 2 nach aussen ausführbar, die die Versteifungsschicht 3 auf den Aussenschlauch 4 drückt und somit versteift. Die Wahl der Materialien und Funktionsweise ist ansonsten analog zu Figur 1.

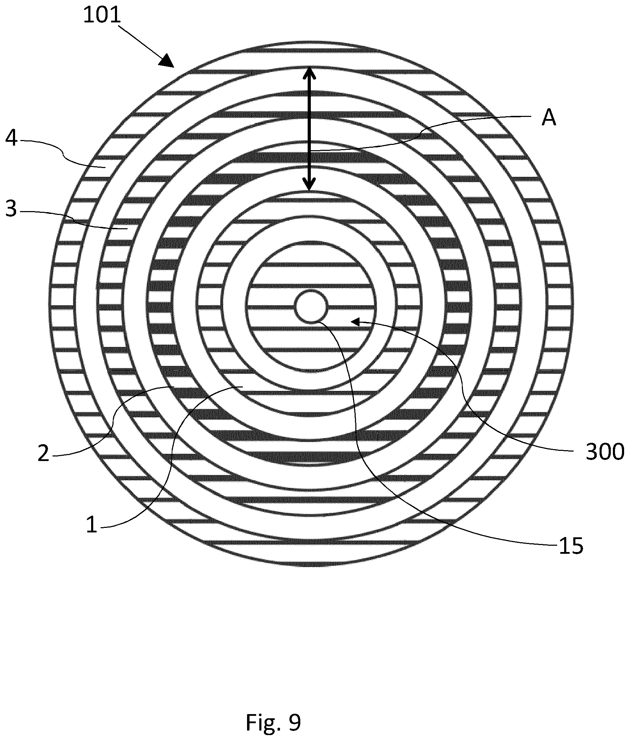

Figur 9 zeigt den Querschnitt des Versteifungsabschnitts 101 mit den vier Schlauchschichten 1, 2, 3, 4 analog zu Figur 8. Zusätzlich weist Figur 9 einen eingeführten weichen Dilatator 300, der ein Lumen 15 für den Führungsdraht 12, sowie zum Ein- und Abführen von Substanzen und/oder Werkzeugen aus/zu den Gefässen aufweist. In dem Bereich A zwischen Innen- und Aussenschlauch 1, 4 kann durch Druck eine radiale Verschiebung des Druckschlauchs 2 und der Versteifungsschicht 3 zum Versteifen des Versteifungsabschnitts 101 stattfinden. Bei dem Versteifungsprozess wird der Druck im Zwischenraum zwischen Innenschlauch 1 und Druckschlauch 2 so stark erhöht, dass sich der Druckschlauch 2 radial bewegt und die Versteifungsschicht 3 gegen den Aussenschlauch 4 drückt.

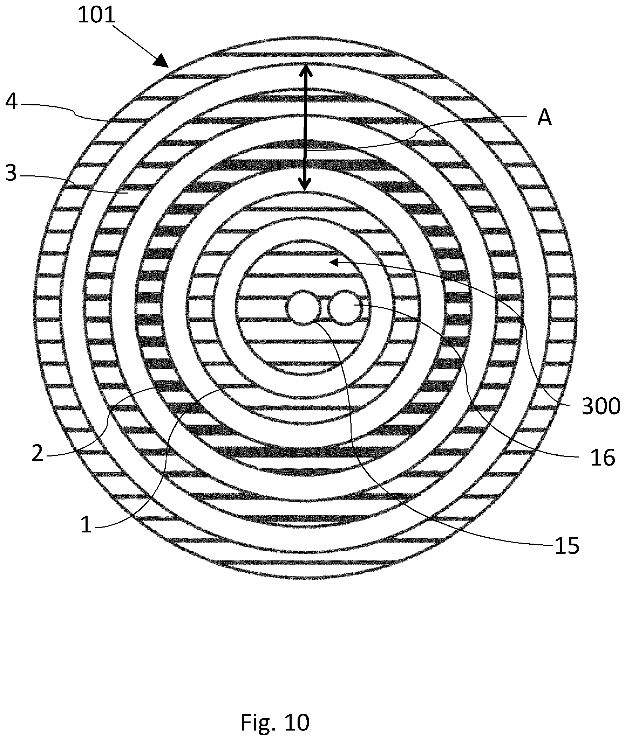

Figur 10 zeigt den Querschnitt des Versteifungsabschnitts 101 analog zu Figur 9, wobei der eingeführte weiche Dilatator 300 das Lumen 15 für den Führungsdraht 12, sowie ein Lumen 16 für den Formdraht 14 aufweist. Das Lumen 15 für den Führungsdraht 12 ist weiterhin zentrisch im weichen Dilatator 300 angeordnet, während das Lumen 16 des Formdrahts 14 zu einer Seite versetzt ist. Die Führung durch die Gefässe mit dem Führungsdraht 12 ist somit weiterhin durch die radialsymmetrische Form gewährleistet, wobei der Formdraht 14 die Form des weichen Dilatators 300 zusätzlich anpassen kann.

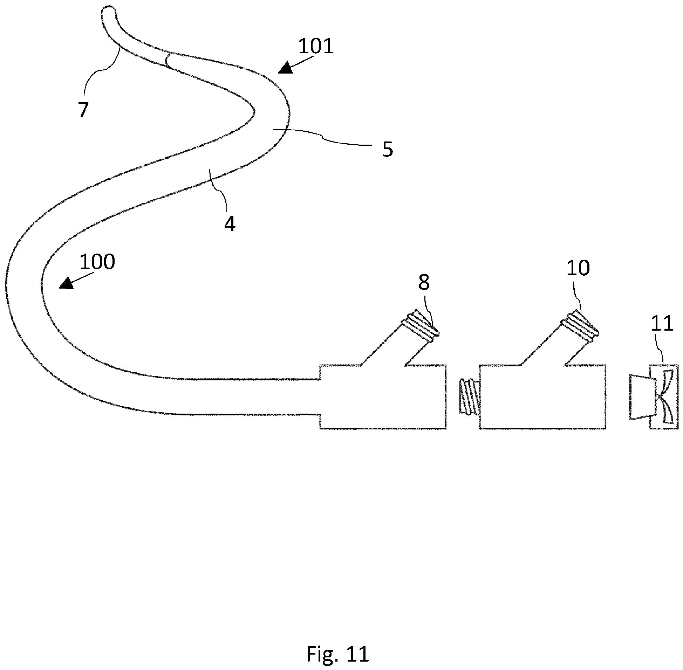

Figur 11 zeigt die Schlauchvorrichtung 100 mit einem Versteifungsabschnitt 101. Die Schlauchvorrichtung 100 zeigt einen konischen vorderen Bereich 7, sodass die Schlauchvorrichtung 101 leichter und atraumatisch zum Eindringen in Gefässe verwendet werden kann. Die Fluidkupplung 8 dient dem Zuführen von Fluid und dem Versteifen des Versteifungsabschnitts 101 der Schlauchvorrichtung 100. Zudem ist ein Seitenanschluss 10 dargestellt, der dem Zuführen von Flüssigkeiten dient. Ein hämostatisches Ventil 11 verhindert zudem, dass Blut aus der Schlauchvorrichtung 100 austreten kann und dennoch Vorrichtungen eingeführt werden können. Der Aussenschlauch 4 weist zudem eine hydrophile Beschichtung 5 auf.

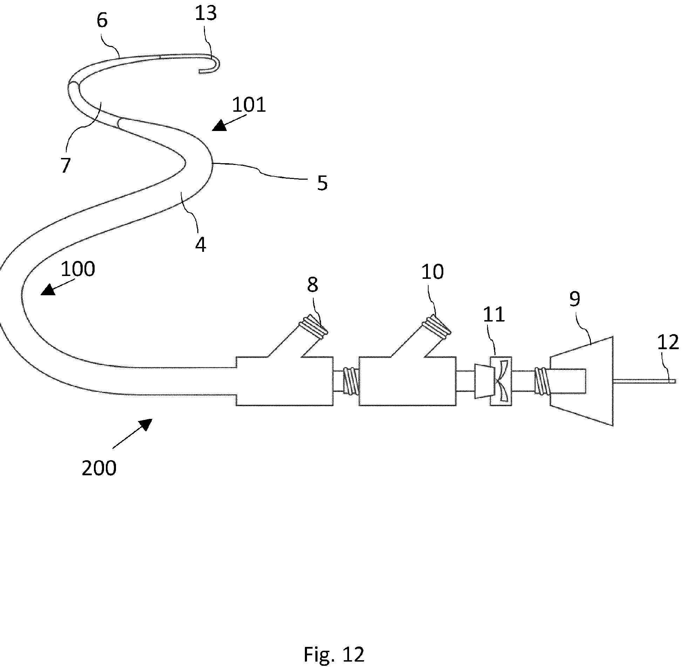

Figur 12 zeigt das Set 200 einer Schlauchvorrichtung 100 und einem Versteifungsabschnitts 101 mit einem Umfang, der vorzugsweise mindestens im Set enthalten ist.
Figur 12 zeigt den Führungsdraht 12, der ein gebogenes vorderes Ende 13 zum Führen der Schlauchvorrichtung 100 aufweist. Des Weiteren ist ein konischer langer aus sehr elastischem Polysiloxan geformte Spitze 6 eines weichen Dilatators 300 zu sehen, der als Führungsvorrichtung verwendet werden kann. Zudem ist der vordere Bereich 7 der Schlauchvorrichtung 100 konisch ausgebildet, damit die Schlauchvorrichtung 100 leichter einzuführen ist und vorzugsweise ebenfalls leichter um Windungen in Gefässen geführt werden kann. Dies wird zusätzlich erleichtert durch eine hydrophile Beschichtung 5 auf dem Aussenschlauch 4.
Des Weiteren ist eine Fluidkupplung 8 in Figur 12 zu sehen, die das Zuführen eines Fluids, vorzugsweise einer Kochsalzlösung, ermöglicht.
In Figur 12 ist noch das Führungselement 9 des weichen Dilatators 300, was für die Steuerung und Translation der Spitze des Dilatators 6 genutzt werden kann, zu sehen. Ein Seitenanschluss 10 ermöglicht zudem, dass Flüssigkeiten zugeführt werden können, wie beispielsweise eine Kontrastlösung, und verschliesst gleichzeitig die Schlauchvorrichtung 100.

Ausserdem weist die Schlauchvorrichtung 100 des Sets ein hämostatisches Ventil 11 auf, das das Set 200 verschliesst und das Einführen von Geräten, Werkzeugen und/oder Kleinstteilen ermöglicht, aber das Austreten von Blut verhindert. In diesem Ausführungsbeispiel umfassen die Schlauchvorrichtung 100 und der weiche Dilatator 300 Eisenstaub, sodass die Bewegungen durch Röntgenverfahren sichtbar gemacht werden können.

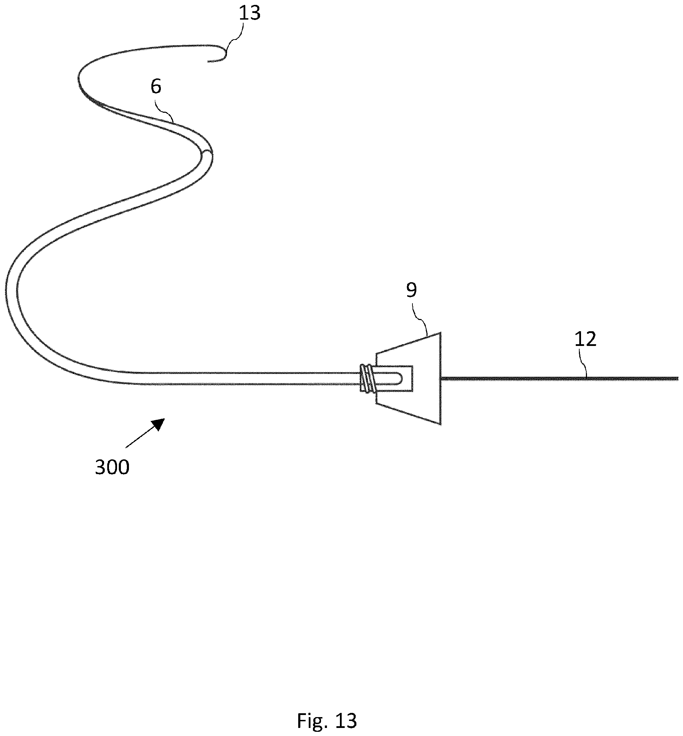

Figur 13 zeigt den weichen Dilatator 300 mit einem eingeführten Führungsdraht 12. Das vordere Ende des Führungsdrahts 12 weist eine Biegung 13 auf. Die Spitze des Dilatators 6 ist konisch zulaufen ausgebildet und kann somit leicht manövriert und eingeführt werden. Es ist hierbei anzumerken, dass der Führungsdraht 12 unabhängig vom weichen Dilatator 300 bewegbar ist und somit den Weg in ein Gefäss vorgeben kann. Das Führungselement 9 des weichen Dilatators 300 ist in diesem Zusammenhang zum Bewegen in longitudinale Richtung des weichen Dilatators 300 verwendbar. Der weiche Dilatator 300 kann somit entlang der vorgegebenen Richtung des Führungsdrahtes 12 geschoben werden.

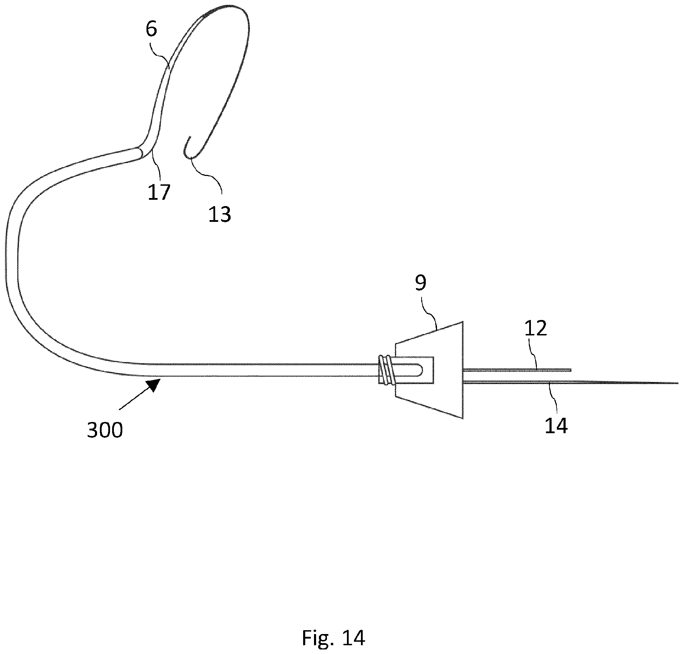

Figur 14 zeigt den weichen Dilatator 300 mit eingeführten Führungsdraht 12, sowie eingeführten Formdraht 14. Dabei ist der Formdraht 14 gemäss Figur 10 seitlich in ein zweites abgetrenntes Lumen 16 einführbar, sodass das Lumen 15 des Führungsdrahtes 12 weiterhin mittig im Dilatator 6 angeordnet werden kann. Die Spitze des Dilatators 6 ist mit Hilfe des Führungselements 9 gemäss den vorgegebenen Biegungen 13 des Führungsdrahtes 12, sowie Dilatatorverformung 17 durch den Formdraht 14 in den Gefässen steuerbar und kann entlang der durch die Drähte 12, 14 vorgegebenen Richtungen bewegt und/oder versteift werden.

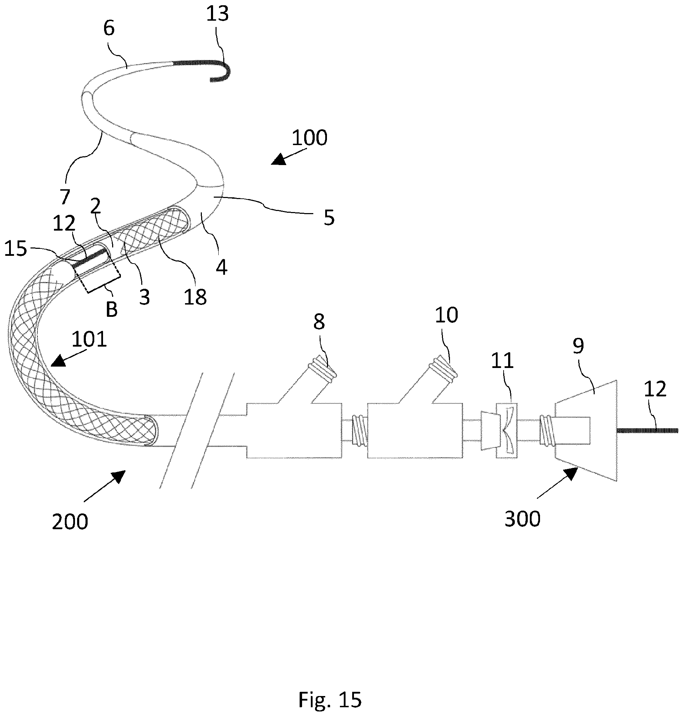

Figur 15 zeigt das Set 200 der Schlauchvorrichtung 100 mit einem Versteifungsabschnitt 101 analog zu Figur 12, wobei analoge Bezeichnungen nicht erneut ausgeführt worden sind. Der Aussenschlauch 4 wurde zur besseren Anschauung partiell entfernt, damit die Versteifungsschicht 3 und Stabilisierungselemente 18 sowie der Druckschlauch 2 darunter, zu sehen sind. Zudem ist ein Abschnitt B des Versteifungsabschnitts 101 mit zusätzlich entfernter Versteifungsschicht 3 und Druckschlauch 2 dargestellt, sodass der Führungsdraht 12 im Lumen 15 des weichen Dilatators 300 sichtbar ist.

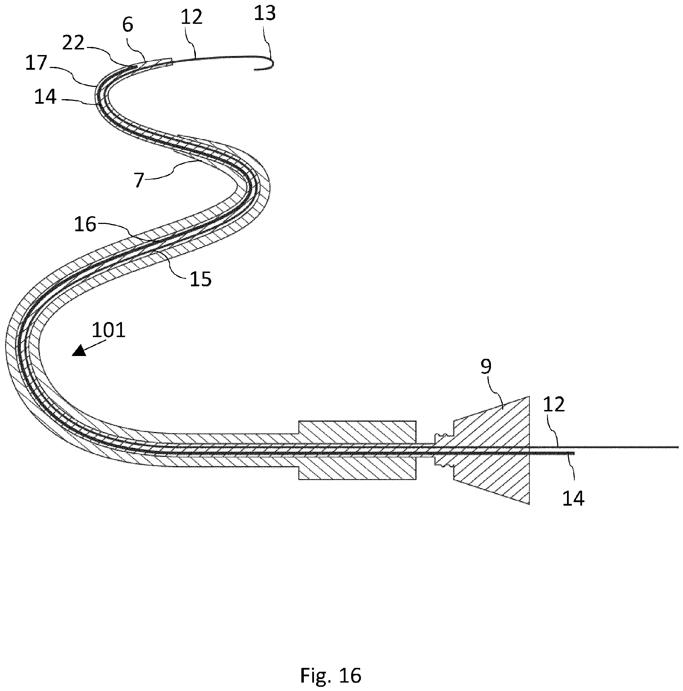

Figur 16 zeigt einen weichen Dilatator 300 mit einem Lumen 15 für den Führungsdraht 12 und einem Lumen 16 für den Formdraht 14. Zur besseren Anschauung des Ausführungsbeispiels wurden die zusätzlichen Schichten einer erfindungsgemässen Schlauchvorrichtung 100 mit Versteifungsabschnitt 101 in diesem Längsschnitt weg gelassen.
Der Längsschnitt zeigt, dass die Spitze des Dilatators 6, die aus dem konischen vorderen Bereich 7 der Schlauchvorrichtung 100 ragt. Die Spitze des Dilatators 6 ist durch den Formdraht 14 zu einer Dilatatorverformung 17 geformt. Der Formdraht 14 kann nicht genug Kraft ausüben, um die Schlauchvorrichtung beim Ein- und Ausführen zu formen, sondern die Verformung in eine Dilatatorverformung 17 tritt erst an der Spitze des Dilatators 6 ein. Zudem zeigt Figur 16, dass das Lumen 16 für den Formdraht 14 ein abgeschlossenes Ende aufweist 22. Somit kann der Formdraht 14 eine Dilatatorverformung 17 vorgeben, die auch vorliegt, wenn der Führungsdraht 12 entfernt werden muss. Des Weiteren kann durch die Dilatatorverformung 17 des Formdrahts 14 in Kombination mit der vorgegebenen Biegung 13 des Führungsdrahts 12 eine praktikable Führung der Schlauchvorrichtung 101 in Gefässen gewährleistet werden.

## Patentansprüche

1. Schlauchvorrichtung (100), insbesondere Schleusenvorrichtung zum Einführen in einen Körpergang, umfassend einen Versteifungsabschnitt (101) umfassend einen Innenschlauch (1), eine Versteifungsschicht (3) und einen Aussenschlauch (4), wobei die Versteifungsschicht (3) konzentrisch ausserhalb des Innenschlauches (1) angeordnet ist und der Aussenschlauch (4) konzentrisch ausserhalb der Versteifungsschicht (3) angeordnet ist, wobei der Innenschlauch (1) und der Aussenschlauch (4) radial steif ausgebildet sind und die Versteifungsschicht (3) radial bewegbar, ausgebildet ist, **dadurch gekennzeichnet, dass** zwischen Innenschlauch (1) und Versteifungsschicht (3) derartig ein Druck aufbaubar ist, dass die Versteifungsschicht (3) von innen gegen den Aussenschlauch (4) drückbar ist und so unter Druck den Versteifungsabschnitt (101) versteift.

2. Schlauchvorrichtung (100) nach Anspruch 1, **dadurch gekennzeichnet, dass** zwischen Innenschlauch (1) und Versteifungsschicht (3) ein erweiterbarer, insbesondere elastischer und/oder auffaltbarer, Druckschlauch (2) angeordnet ist.

3. Schlauchvorrichtung (100) nach einem der vorhergehenden Ansprüchen, **dadurch gekennzeichnet, dass** die Versteifungsschicht (3) zumindest zwei Stabilisierungselemente (18) umfasst, wobei die Ausrichtung der Stabilisierungselemente (18) zumindest einen Teilvektor in Längsrichtung der Schlauchvorrichtung aufweist und die Stabilisierungselemente (18) vorzugsweise gegeneinander verschiebbar sind.

4. Schlauchvorrichtung (100) nach Anspruch 3, **dadurch gekennzeichnet, dass** die Stabilisierungselemente (18) aus Metall und/oder Kunststoff bestehen und insbesondere eine Vielzahl von Stabilisierungselementen (18) zu einem Geflecht verflochten ist.

5. Schlauchvorrichtung (100) nach einem der vorhergehenden Ansprüche, **dadurch gekennzeichnet, dass** der Innenschlauch (1) und der Aussenschlauch (4) druckdicht ausgebildet sind und insbesondere aus einem Kunststoff und/oder Metall hergestellt sind.

6. Schlauchvorrichtung (100) nach einem der vorhergehenden Ansprüche, **dadurch gekennzeichnet, dass** die Schlauchvorrichtung (100) eine Fluidkupplung (8) umfasst, durch die ein Fluid in den Bereich zwischen Innenschlauch (1) und Versteifungsschicht (3), insbesondere in den Druckschlauch (2), einbringbar ist.

7. Schlauchvorrichtung (100) nach einem der vorhergehenden Ansprüche, **dadurch gekennzeichnet, dass** die Schlauchvorrichtung (100) zumindest teilweise ein im Röntgen sichtbares Material, insbesondere Metall, umfasst.

8. Schlauchvorrichtung (100) nach einem der vorhergehenden Ansprüche, **dadurch gekennzeichnet, dass** ein vorderer Bereich (7) der Schlauchvorrichtung (100) konisch ausgebildet ist.

9. Schlauchvorrichtung (100) nach einem der vorhergehenden Ansprüche, **dadurch gekennzeichnet, dass** aussen auf dem Aussenschlauch (4) eine hydrophile oder hydrophobe Beschichtung (5) angeordnet ist.

10. Schlauchvorrichtung (100) nach einem der vorhergehenden Ansprüche, **dadurch gekennzeichnet, dass** der Versteifungsabschnitt (101) einen Teil oder die gesamte Schlauchvorrichtung (100) bildet.

11. Schlauchvorrichtung (100) nach einem der vorhergehenden Ansprüche **dadurch gekennzeichnet, dass** der Innenschlauch (1) einen Innendurchmesser von 1 bis 11,333 mm aufweist.

12. Schlauchvorrichtung (100) nach einem der vorhergehenden Ansprüche **dadurch gekennzeichnet, dass** der Innenschlauch (1) der Schlauchvorrichtung (100) mindestens zwei Lumina aufweist, sodass mehrere Vorrichtungen in getrennten Lumina durch die Schlauchvorrichtung (100) eingeführt werden können.

13. Set umfassend eine Schlauchvorrichtung (100) nach einem der vorhergehenden Ansprüche und mindestens einen Dilatator, sowie insbesondere mindestens einen weichen Dilatator (300) und einen Formdraht (14) und/oder Führungsdraht (12).

14. Verfahren zum selektiven Versteifen einer Schlauchvorrichtung (100) nach einem der vorhergehenden Ansprüche, **dadurch gekennzeichnet, dass** durch Einbringen eines Fluids in den Zwischenraum zwischen Innenschlauch (1) und Versteifungsschicht (3) die Schlauchvorrichtung (100) versteift wird.

15. Verfahren nach Anspruch 11, **dadurch gekennzeichnet, dass** durch Reduktion des Druckes im Zwischenraum zwischen Innenschlauch (1) und Versteifungsschicht (3) die Schlauchvorrichtung (100) wieder beweglich wird.

16. Ein weicher Dilatator, der zwei Lumina umfasst, wobei ein Lumen (15) für den Führungsdraht (12) und ein Lumen (16) für den Formdraht (14) vorgesehen ist, **dadurch gekennzeichnet, dass** das Lumen (16) für den Formdraht (14) in der Spitze des Dilatators (6) abgeschlossen ist und das Lumen (15) für den Führungsdraht (12) offen ist, wobei der weiche Dilatator (300) vorzugsweise hydrophil beschichtet ist, der weiche Dilatator (300) bevorzugt weicher als der Formdraht (14) ist, und der weiche Dilatator (6) vorzugsweise im Röntgen sichtbar ist.
